# FASCICULE DE BREVET EUROPEEN

(11) **EP 0 359 620 B1**
(45) Date de publication et mention de la délivrance du brevet: **28.07.1993**
(21) Numéro de dépôt: 89402386.0
(22) Date de dépôt: 01.09.1989
(51) Int. Cl.: F17C 7/00, A61K 7/00

(54) **Procédé et récipient pour fournir du CO2 supercritique**
Verfahren und Behälter zum Abliefern vom superkritischen Kohlendioxid
Process and container for delivering supercritical carbon dioxide

(30) Priorité: 08.09.1988 FR 8811738
(43) Date de publication de la demande: 21.03.1990
(73) Titulaire: L'AIR LIQUIDE, SOCIETE ANONYME POUR L'ETUDE ET L'EXPLOITATION DES PROCEDES GEORGES CLAUDE, 75321 Paris Cédex 07 (FR); CARBOXYQUE FRANCAISE, F-92800 Puteaux (FR)
(72) Inventeur: Frejaville, Serge, F-30340 Mejannes-les-Ales (FR); Mittelman, Philippe, F-94260 Fresnes (FR); Rajaonarivello, Claude, F-94130 Nogent-sur-Marne (FR); Naud, Jean-Michel, F-92000 Nanterre (FR)
(74) Mandataire: Le Moenner, Gabriel

(56) Documents cités:
- EP-A- 0 235 017
- EP-A- 0 295 169
- GB-A- 621 019

## Description

La présente invention est relative à un procédé de fourniture d'anhydride carbonique supercritique, utilisable par exemple dans l'industrie cosmétique en tant qu'agent solvant et propulseur.

La technique courante pour fournir de l'anhydride carbonique supercritique consiste à utiliser un stockage d'anhydride carbonique liquide à - 20°C sous 20 bars avec une pompe haute pression à plusieurs étages et un groupe frigorifique, ce qui est coûteux et compliqué.

Le document GB-A-621.019 a proposé de constituer, dans un récipient pourvu de moyens de soutirage, une charge d'anhydride carbonique surmontée d'une charge gazeuse sous pression élevée, et cela dans le but de procéder à une décharge rapide à des températures même très basses, de l'ordre de - 50°C sans cependant conduire à des pressions excessives à des températures aussi élevées que + 60°C. Le but essentiel d'une telle charge dans un récipient selon ce document est de constituer un système d'extinction d'incendie susceptible d'opérer correctement à n'importe quelle température ambiante. A cet effet, on choisit un gaz qui reste à l'état gazeux même à température très basse et la document cite les principaux gaz remplissant cette condition.

La présente invention vise à délivrer de l'anhydride carbonique supercritique de façon simple et économique et cet objectif est atteint en ce qu'on constitue ladite charge gazeuse par de l'hydrogène sous une pression supérieure à 120 bars environ et en ce que ladite fourniture d'anhydride carbonique supercritique est effectuée par soutirage en maintenant une pression de la charge gazeuse d'hydrogène qui ne soit jamais inférieure à 120 bars environ.

Selon une forme de mise en oeuvre, la charge gazeuse d'hydrogène, initialement à une pression nettement supérieure à 120 bars environ, est maintenue en l'état pendant la fourniture d'anhydride carbonique, et on cesse tout soutirage d'anhydride carbonique dès que la pression de ladite charge gazeuse d'hydrogène s'est réduite du fait du soutirage d'anhydride carbonique liquide à une valeur de l'ordre de 120 bars environ et avantageusement, pour éviter à coup sûr que la pression de soutirage ne descende en dessous de 120 bars environ, on soutire l'anhydride carbonique liquide au moyen d'un tube plongeur, le volume de la charge de l'anhydride carbonique liquide et la longueur de ce tube étant choisis de façon que la pression de l'hydrogène ne descende pas au-dessous de 120 bars environ en fin de soutirage de l'anhydride carbonique. On peut également soutirer l'anhydride carbonique liquide à travers une conduite munie d'une vanne pressostatique réglée à fermeture automatique pour toute valeur de la pression inférieure à 120 bars environ.

Selon une autre forme de mise en oeuvre, on introduit une charge d'anhydride carbonique liquide dans un premier récipient résistant à la pression pourvu de moyens de soutirage liquide, on met la partie supérieure du récipient en communication avec un récipient auxiliaire contenant de l'hydrogène de manière à fournir de l'hydrogène au premier récipient sous une pression non inférieure à 120 bars environ, et l'on maintient cette communication pendant le soutirage de l'anhydride carbonique liquide.

L'invention a également pour objet un équipement de stockage et de fourniture d'anhydride carbonique liquide qui comprend un récipient pourvu de moyens de soutirage de liquide avec une charge d'anhydride carbonique liquide contenant 7 à 8 % environ d'hydrogène dissous, surmontée d'un ciel gazeux constitué d'hydrogène sous une pression supérieure à 120 bars environ.

Selon une forme de réalisation, la pression du ciel gazeux constitué d'hydrogène est d'environ 160 bars à + 20°C.

Selon une autre forme de réalisation, le récipient contient une conduite de soutirage pourvue d'une vanne pressostatique à fermeture pour toute pression inférieure à 120 bars environ.

Selon encore une autre forme de réalisation, la partie supérieure du récipient communique avec un récipient auxiliaire contenant de l'hydrogène sous une pression non inférieure à 120 bars environ et de préférence les deux récipients sont reliés par une conduite équipée d'un détendeur.

Des exemples de mise en oeuvre de l'invention vont maintenant être décrits en regard des dessins annexés, sur lesquels :
- les figures 1 et 2 illustrent deux variantes du procédé suivant l'invention ;
- la figure 3 est une vue en élévation, partiellement en coupe, d'un équipement conforme à l'invention ; et
- la figure 4 représente en coupe longitudinale un détail de l'équipement de la figure 3.

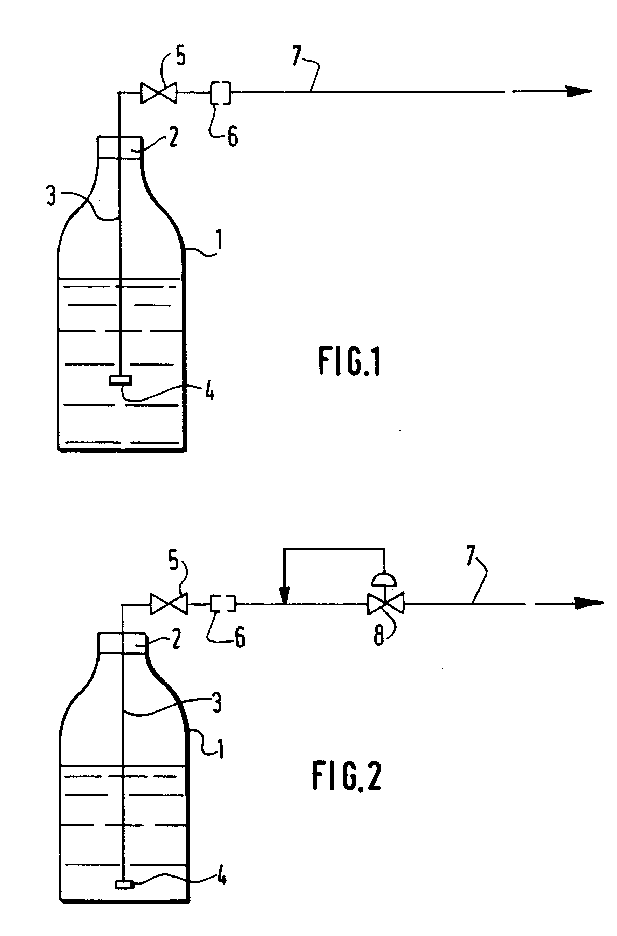

On a représenté à la figure 1, en coupe longitudinale, une bouteille 1 en acier, dont le col est équipé d'un bouchon 2 traversé par un tube plongeur 3, lequel comporte à son extrémité inférieure un orifice calibré 4. Le tube 3 descend jusqu'à un niveau situé nettement au-dessus du fond de la bouteille et qui sera défini plus loin.

A l'extérieur de la bouteille, le tube 3 est pourvu d'un robinet d'arrêt 5 et se termine par un raccord 6 pour le raccordement d'une conduite d'utilisation 7.

La bouteille 1 est remplie de la façon suivante.

Après avoir effectué un traitement préalable classique pour les bouteilles destinées à recevoir des gaz très purs, on introduit dans la bouteille par le tube 3 une charge de C0₂ liquide supercritique en provenance d'un stockage à - 20°C, 20 bars, au moyen d'une pompe de circulation et d'un réchauffeur qui l'amène aux environs de 0°C, ce pour de raisons liées à la résilience de l'acier dont est constituée la bouteille 1. Le CO₂ a une pureté de 99.998 % et, par pesée de la bouteille, on en mesure une quantité donnée. Cette quantité, qui doit être suffisante pour que l'orifice calibré 4 soit immergé, correspond par exemple à un volume de liquide égal à environ la moitié du volume de la bouteille.

On laisse ensuite la bouteille revenir à la température ambiante, ce qui constitue une phase de stabilisation du CO₂ qui dure au moins deux heures.

Puis on dispose la bouteille tête en bas, par tout moyen de manipulation approprié. L'orifice calibré 4 émerge alors du liquide, et on attend, pendant une nouvelle phase de stabilisation de l'ordre de 10 min, que le liquide se soit bien rassemblé et que l'équilibre liquide-vapeur soit de nouveau atteint.

On introduit alors par le tube 3 une charge d'hydrogène à haute pureté (moins de 5 ppm d'eau, moins de 5 ppm d'oxygène), jusqu'à atteindre une pression de 160 bars environ. Cette pression est choisie aussi haute que possible mais telle que, pour la température ambiante maximale prévisible (par exemple pour + 50°C), elle reste au-dessous de la pression de service de la bouteille 1.

Enfin, on remet la bouteille tête en haut et, après un nouveau temps de stabilisation de l'ordre de une heure, elle est prête à fournir du CO₂ supercritique par simple ouverture du robinet 5.

La bouteille se trouve alors dans l'état représenté à la figure 1, c'est-à-dire qu'elle contient une charge constituée :
- d'une masse de CO₂ liquide supercritique contenant une certaine quantité d'hydrogène dissous, et
- d'un ciel gazeux sous 160 bars environ, constitué uniquement d'hydrogène.

Les analyses ont montré que lorsque la pression de l'hydrogène décroît de 160 bars à 120 bars environ, la teneur en hydrogène dissous du CO₂ liquide est faible et se maintient à une valeur sensiblement constante comprise entre 7 et 8 %, alors que cette teneur croît brusquement lorsque la pression descend au-dessous de 120 bars environ. Par conséquent, si l'on veut ne soutirer que du CO₂ à faible teneur en hydrogène, on arrêtera le soutirage lorsque la pression, qui décroît au fur à mesure de la baisse du niveau du liquide, atteint une valeur limite non inférieure à cette valeur de 120 bars environ.

Pour cela, on peut, comme représenté à la figure 1, ne faire descendre le tube 3 que jusqu'à un niveau correspondant au volume du ciel gazeux qui fournit la pression de 120 bars environ. Si le volume de remplissage du CO₂ liquide est choisi à l'avance, par exemple égal à peu près à la moitié de la capacité de la bouteille comme indiqué plus haut, on en déduit par calcul le niveau en question. En variante, si l'on préfère soutirer la quasi-totalité du liquide, le tube 3 descendra presque jusqu'au fond de la bouteille, et l'on calculera le volume initial du CO₂ liquide de façon correspondante.

Cependant, dans ces deux cas, la conduite 7 véhiculera un mélange gazeux CO₂-hydrogène, ou de l'hydrogène pur, en fin de soutirage. Pour éviter ceci, on peut faire appel au mode de mise en oeuvre illustré à la figure 2 : la bouteille contenant initialement à peu près la moitié de son volume en CO₂ liquide, le tube 3 descend presque jusqu'au fond de la bouteille, et la conduite d'utilisation 7 est équipée d'une vanne pressostatique 8 qui se ferme lorsque la pression descend à la valeur limite de 120 bars environ. Le soutirage s'arrête ainsi automatiquement, et l'on change alors de bouteille.

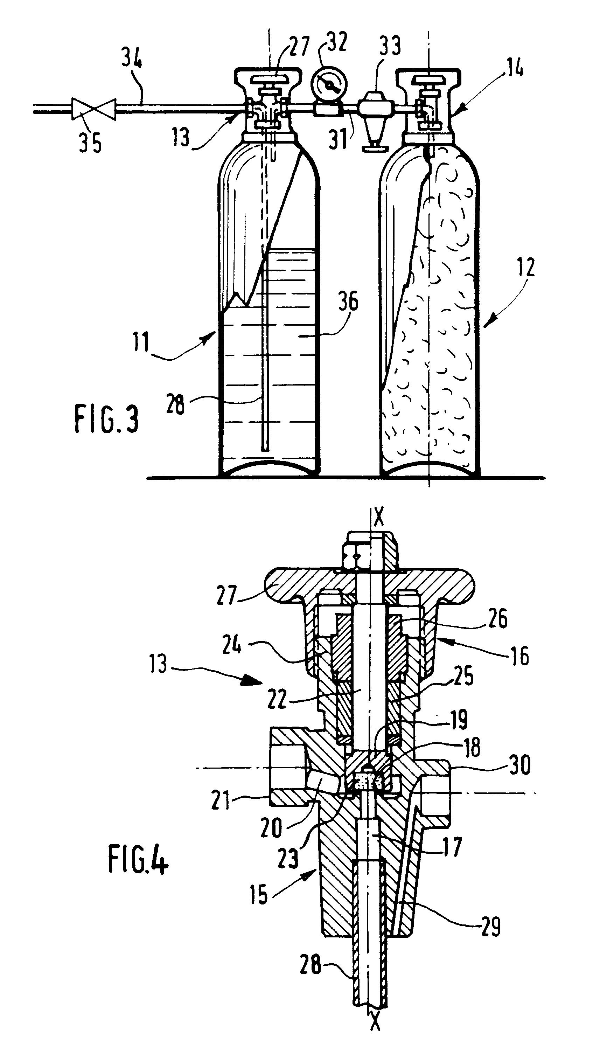

On a représenté à la figure 3 un équipement constitué de deux bouteilles 11 et 12 de mêmes dimensions équipées chacune d'un robinet 13, 14, respectivement.

Le robinet 13, représenté à plus grande échelle à la figure 4, comprend un corps 15, d'axe général X-X, et un dispositif de manoeuvre 16. La partie inférieure du corps 15, fixée dans le col de la bouteille 11, définit un conduit d'entrée 17 qui se termine par un siège 18 et débouche dans une chambre 19. Un conduit de soutirage 20, qui se termine par un raccord principal 21, part latéralement de la chambre 19, et celle-ci présente un orifice supérieur dans lequel coulisse l'extrémité inférieure de la tige de manoeuvre 22 du robinet, laquelle est pourvue d'un clapet 23 pouvant s'appliquer sur le siège 18.

La partie supérieure du corps 15 forme une tubulure 24 qui est coaxiale à la tige 22. Le dispositif de manoeuvre 16 comprend, outre la tige 22, un joint d'étanchéité 25 qui entoure cette tige et qui est comprimé dans la tubulure 24 par un écrou de presse-étoupe 26, ainsi qu'un volant de manoeuvre 27 vissé sur l'extrémité supérieure de la tubulure 24.

Un tube plongeur 28, qui s'étend presque jusqu'au fond de la bouteille 11, est emmanché dans le conduit d'entrée 17, et la partie inférieure du corps 15 comprend un perçage supplémentaire 29 qui met la partie supérieure de la bouteille en communication avec un raccord auxiliaire latéral 30.

Le robinet 14 est identique au robinet 13, à ceci près que son corps est dépourvu du perçage 29 et du raccord auxiliaire 30. De plus, la bouteille 12 ne comporte pas de tube plongeur, de sorte que le conduit d'entrée 17 du robinet 14 débouche directement dans la partie supérieure de cette bouteille.

Comme on le voit à la figure 3, le raccord 30 du robinet 13 est relié au raccord 21 du robinet 14 par une conduite 31 équipée d'un manomètre 32 et d'un détendeur 33. Deplus, une conduite d'utilisation 34 munie d'une vanne d'arrêt 35 est reliée au raccord 21 de la bouteille 11.

Au départ, la bouteille 12 contient de l'hydroègne sous une pression nettement supérieure à 120 bars, par exemple sous 200 bars ; le robinet 14 est fermé, et la bouteille 11 est remplie jusqu'à un niveau prédéterminé de CO₂ liquide, de la manière décrite en regard des figures 1 et 2, à travers la conduite 34.

Puis, la vanne 35 étant fermée, on règle de détendeur 33 sur une pression de sortie P au moins égale à 120 bars environ, et l'on ouvre le robinet 14 de la bouteille 12. Le CO₂ liquide est ainsi pressurisé à la pression P et se trouve à l'état supercritique. La bouteille 11 contient alors une masse 36 de CO₂ liquide supercritique contenant environ 7 à 8 % d'hydrogène dissous, surmontée d'un ciel gazeux constitué uniquement d'hydrogène.

On peut alors soutirer le CO₂ supercritique par la conduite 34, en ouvrant la vanne 35. Au fur et à mesure de la baisse du niveau du CO₂ liquide, de l'hydrogène passe de la bouteille 12 à la bouteille 11, et celle-ci reste constamment à la même pression P. Ceci reste vrai tant que la pression de la bouteille 12 reste supérieure à la valeur P ; par suite, en choisissant convenablement le niveau initial du CO₂ liquide dans la bouteille 11, on peut soutirer la totalité du CO₂ sous cette même pression P.

En variante, on pourrait se passer du détendeur 33. Le CO₂ serait alors délivré sous une pression progressivement décroissante à partir de la pression initiale régnant dans la bouteille 12.

## Revendications

1. Procédé de fourniture d'anhydride carbonique supercritique, du genre où l'on constitue, dans un récipient (1) (11) pourvu de moyens de soutirage (3) (28), une charge d'anhydride carbonique surmontée d'une charge gazeuse sous pression élevée, caractérisé en ce qu'on constitue ladite charge gazeuse par de l'hydrogène sous une pression supérieure à 120 bars environ et en ce que ladite fourniture d'anhydride carbonique supercritique est effectuée par soutirage (3) (28) en maintenant une pression de la charge gazeuse d'hydrogène, qui ne soit pas inférieure à 120 bars environ.

2. Procédé de fourniture d'anhydride carbonique supercritique selon la revendication 1, caractérisé en ce que la charge gazeuse d'hydrogène, initialement à une pression nettement supérieure à 120 bars environ, est maintenue constante pendant la fourniture d'anhydride carbonique, et on cesse tout soutirage d'anhydride carbonique dès que la pression de ladite charge gazeuse d'hydrogène s'est réduite à une valeur de l'ordre de 120 bars environ.

3. Procédé selon la revendication 2, caractérisé en ce que, pour éviter que la pression de soutirage ne descende en dessous de 120 bars environ, on soutire l'anhydride carbonique liquide au moyen d'un tube plongeur (3), le volume de la charge de l'anhydride carbonique liquide et la longueur de ce tube (3) étant choisis de façon que la pression de l'hydrogène ne descende pas au-dessous de 120 bars environ en fin de soutirage de l'anhydride carbonique.

4. Procédé selon la revendication 2, caractérisé en ce qu'on soutire l'anhydride carbonique liquide à travers une conduite (7) munie d'une vanne pressostatique (8) réglée à fermeture automatique pour toute valeur de la pression inférieure à 120 bars environ.

5. Procédé selon l'une quelconque des revendications 2 à 4, caractérisé en ce que le récipient (1) est une bouteille pourvue d'un tube plongeur (3) et en ce qu'on introduit la charge de d'anhydride carbonique par le tube plongeur puis, la bouteille étant disposée tête en bras et après une période de stabilisation de l'anhydride carbonique, on introduit la charge d'hydrogène par le tube plongeur jusqu'à une pression nettement supérieure à 120 bars environ, puis on dispose la bouteille tête en haut en position pour effectuer, après nouvelle stabilisation, le soutirage de l'anhydride carbonique.

6. Procédé selon la revendication 1, caractérisé en ce qu'on introduit une charge (36) d'anhydride carbonique liquide dans un premier récipient (11) résistant à la pression pourvu de moyens (28) de soutirage liquide, on met la partie supérieure du récipient en communication avec un récipient auxiliaire (12) contenant de l'hydrogène de manière à fournir de l'hydrogène au premier récipient (11) sous une pression non inférieure à 120 bars environ, et l'on maintient cette communication pendant le soutirage de l'anhydride carbonique liquide.

7. Procédé selon la revendication 6, caractérisé en ce qu'on détend l'hydrogène à une pression prédéterminée non inférieure à 120 bars environ entre le récipient auxiliaire d'hydrogène (12) et le récipient (11) contenant de l'anhydride carbonique.

8. Equipement de stockage et de fourniture d'anhydride carbonique supercritique, caractérisé en ce qu'il comprend un récipient (1) (11) pourvu de moyens (3) (18) de soutirage de liquide avec une charge d'anhydride carbonique liquide contenant 7 à 8 % environ d'hydrogène dissous, surmontée d'un ciel gazeux constitué d'hydrogène sous une pression supérieure à 120 bars environ.

9. Equipement selon la revendication 8, caractérisé en ce que la pression du ciel gazeux constitué d'hydrogène est d'environ 160 bars à + 20°C.

10. Equipement selon la revendication 9, caractérisé en ce que le récipient contient une conduite de soutirage pourvue d'une vanne pressostatique (8) à fermeture pour toute pression inférieure à 120 bars environ.

11. Equipement selon l'une des revendications 8 et 9, caractérisé en ce que la partie supérieure du récipient (11) communique avec un récipient auxiliaire (12) contenant de l'hydrogène sous une pression supérieure à 120 bars environ.

12. Equipement suivant la revendication 11, caractérisé en ce que les deux récipients (11) (12) sont reliés par une conduite (31) équipée d'un détendeur (33).

## Claims

1. A method for supplying supercritical carbonic anhydride, of the type in which there is constituted, in a container (1) (11) provided with drawing-off means (3) (28), a charge of carbonic anhydride, topped by a gaseous charge under high pressure, characterised in that said gaseous charge is constituted by hydrogen under a pressure greater than approximately 120 bar and in that said supply of supercritical carbonic anhydride is carried out by drawing-off (3) (28), maintaining a pressure of the gaseous hydrogen charge, which is not less than approximately 120 bar.

2. A method for supplying supercritical carbonic anhydride according to Claim 1, characterised in that the gaseous hydrogen charge, initially at a pressure distinctly greater than approximately 120 bar, is kept constant during the supply of carbonic anhydride, and all drawing-off of carbonic anhydride is ceased as soon as the pressure of said gaseous hydrogen charge has reduced to a value In the order of approximately 120 bar.

3. A method according to Claim 2, characterised in that, to prevent the drawing-off pressure from falling below approximately 120 bar, the liquid carbonic anhydride is drawn off by means of a plunger tube (3), the volume of the charge of liquid carbonic anhydride and the length of this tube (3) being selected such that the pressure of the hydrogen does not fall below approximately 120 bars at the end of drawing-off of the carbonic anhydride.

4. A method according to Claim 2, characterised in that the liquid carbonic anhydride is drawn off through a duct (7) provided with a pressostatic valve (8) regulated for automatic closure for any value of pressure lower than approximately 120 bar.

5. A method according to any one of Claims 2 to 4, characterised in that the container (1) is a bottle provided with a plunger tube (3) and in that the charge of carbonic anhydride is introduced through the plunger tube, then, with the bottle being arranged upside down, and after a period of stabilisation of the carbonic anhydride, the hydrogen charge is introduced through the plunger tube up to a pressure distinctly greater than approximately 120 bar, then the bottle is arranged upright in a position to carry out, after renewed stabilisation, the drawing-off of the carbonic anhydride.

6. A method according to Claim 1, characterised in that a charge (36) of liquid carbonic anhydride is introduced into a first container (11) resistant to pressure, provided with means (28) for liquid drawing-off, the upper part of the container is put in communication with an auxiliary container (12) containing hydrogen so as to supply hydrogen to the first container (11) under a pressure not less than approximately 120 bar, and this communication is maintained during the drawing-off of the liquid carbonic anhydride.

7. A method according to Claim 6, characterised in that the hydrogen is expanded at a predetermined pressure not less than approximately 120 bars between the auxiliary hydrogen container (12) and the container (11) containing carbonic anhydride.

8. Equipment for the storage and supply of supercritical carbonic anhydride, characterised in that it comprises a container (1) (11) provided with means (3) (18) for drawing-off of liquid with a charge of liquid carbonic anhydride containing approximately 7 to 8 % dissolved hydrogen, topped by a gaseous covering constituted of hydrogen under a pressure greater than approximately 120 bar.

9. Equipment according to Claim 8, characterised in that the pressure of the gaseous covering constituted of hydrogen is approximately 160 bar at + 20°C.

10. Equipment according to Claim 9, characterised in that the container contains a drawing-off duct provided with a pressostatic valve (8), with closure for any pressure less than approximately 120 bar.

11. Equipment according to one of Claims 8 and 9, characterised in that the upper part of the container (11) communicates with an auxiliary container (12) containing hydrogen at a pressure greater than approximately 120 bar.

12. Equipment according to Claim 11, characterised in that the two containers (11) (12) are connected by a duct (31) equipped with a relief valve (33).

## Patentansprüche

1. Verfahren zur Lieferung von überkritischem Kohlendioxid, bei dem man in einem Behälter (1) (11), der mit Mitteln (3) (28) zum Abziehen ausgestattet ist, eine Charge von Kohlendioxid bildet, die unter einer gasförmigen Charge unter erhöhtem Druck angeordnet ist, dadurch gekennzeichnet, daß man diese gasförmige Charge aus Wasserstoff unter einem höheren Druck als ungefähr 120 bar bildet und daß diese Lieferung von überkritischem Kohlendioxid durch Abziehen (3) (28) erfolgt, wobei ein Druck der gasförmigen Wasserstoffcharge aufrechterhalten wird, der nicht niedriger als ungefähr 120 bar ist.

2. Verfahren zur Lieferung von überkritischem Kohlendioxid nach Anspruch 1, dadurch gekennzeichnet, daß die gasförmige Wasserstoffcharge, die anfangs einen Druck deutlich höher als ungefähr 120 bar hat, konstant während der Lieferung von Kohlendioxid gehalten wird und man jedes Abziehen von Kohlendioxid einstellt, sobald sich der Druck dieser gasförmigen Wasserstoffcharge auf einen Wert der Größenordnung von ungefähr 120 bar reduziert hat.

3. Verfahren nach Anspruch 2, dadurch gekennzeichnet, daß man, um zu vermeiden, daß der Druck des Abziehens nicht unter ungefähr 120 bar sinkt, flüssiges Kohlendioxid mittels eines Tauchrohres (3) abzieht, wobei das Volumen der flüssigen Kohlendioxidcharge und die Länge dieses Rohres (3) derart gewählt werden, daß der Wasserstoffdruck am Ende des Abziehens des Kohlendioxid nicht unter ungefähr 120 bar sinkt.

4. Verfahren nach Anspruch 2, dadurch gekennzeichnet, daß man flüssiges Kohlendioxid durch eine Leitung (7) abzieht, die mit einem pressostatischen Ventil (8) versehen ist, welches mit automatischem Verschluß für jeden Druckwert unterhalb von ungefähr 120 bar reguliert wird.

5. Verfahren nach einem der Ansprüche 2 bis 4, dadurch gekennzeichnet, daß der Behälter (1) eine mit einem Tauchrohr (3) versehene Flasche ist und daß man die Kohlendioxidcharge durch das Tauchrohr einführt, dann, wenn die Flasche Kopf nach unten gelagert ist und nach einer Stabilisierungsperiode des Kohlendioxid, man die Wasserstoffcharge durch das Tauchrohr bis zu einem Druck deutlich über ungefähr 120 bar einführt, und man dann die Flasche mit Kopf nach oben in die Stellung bringt, um nach neuer Stabilisierung das Abziehen des Kohlendioxid durchzuführen.

6. Verfahren nach Anspruch 1, dadurch gekennzeichnet, daß man eine flüssige Kohlendioxidbeschickung (36) in einen ersten druckbeständigen Behälter (1) einführt, der mit Mitteln (28) für das flüssige Abziehen versehen ist, man den oberen Teil des Behälters mit einem Hilfsbehälter (12) in Verbindung bringt, der Wasserstoff der Art enthält, daß er dem ersten Behälter (11) Wasserstoff unter einem Druck nicht unter ungefähr 120 bar liefert, und daß man diese Verbindung während des Abziehens des flüssigen Kohlendioxids aufrechterhält.

7. Verfahren nach Anspruch 6, dadurch gekennzeichnet, daß man den Wasserstoff auf einem vorbestimmten Druck nicht niedriger als ungefähr 120 bar zwischen dem Hilfsbehälter (12) für Wasserstoff und dem Behälter (11), der das Kohlendioxid enthält, entspannt.

8. Einrichtung für die Speicherung und die Lieferung von überkritischem Kohlendioxid, dadurch gekennzeichnet, daß sie einen Behälter (1) (11) aufweist, der mit Mitteln (3) (18) zum Abziehen von Flüssigkeit versehen ist, mit einer flüssigen Kohlendioxidcharge, die ungefähr 7 bis 8 % gelösten Wasserstoff enthält, welche unter einer gasförmigen Schicht, die aus Wasserstoff unter einem Druck höher als ungefähr 120 bar besteht, angeordnet ist.

9. Einrichtung nach Anspruch 8, dadurch gekennzeichnet, daß der Druck der gasförmigen, aus Wasserstoff bestehenden Schicht ungefähr 160 bar bei + 20°C beträgt.

10. Einrichtung nach Anspruch 9, dadurch gekennzeichnet, daß der Behälter eine Abzugsleitung enthält, die mit einem pressostatischen Ventil (8) mit Schließung für jeden Druck unterhalb von ungefähr 120 bar versehen ist.

11. Einrichtung nach einem der Ansprüche 8 und 9, dadurch gekennzeichnet, daß der obere Teil des Behälters (11) mit einem Hilfsbehälter (12) in Verbindung steht, welcher Wasserstoff unter einem höheren Druck als ungefähr 120 bar enthält.

12. Einrichtung nach Anspruch 11, dadurch gekennzeichnet, daß die beiden Behälter (11) (12) durch eine Leitung (31) verbunden sind, welche mit einer Entspannungseinrichtung (33) ausgestattet ist.
